# EUROPÄISCHE PATENTSCHRIFT

(11) **EP 2 106 751 B1**
(45) Veröffentlichungstag und Bekanntmachung des Hinweises auf die Patenterteilung: **20.12.2017**
(21) Anmeldenummer: 09156415.3
(22) Anmeldetag: 27.03.2009
(51) Int. Cl.: A61B 17/00, A61B 17/29

(54) **Medizinisches Instrument mit arretierbarer Abwinkelsteuerung**
Medical instrument with adjustable angle control
Instrument médical doté d'une commande de pliage pouvant être bloquée

(30) Priorität: 31.03.2008 DE 102008017300
(43) Veröffentlichungstag der Anmeldung: 07.10.2009
(73) Patentinhaber: Karl Storz GmbH & Co. KG, 78532 Tuttlingen (DE)
(72) Erfinder: Bacher, Uwe, 78532 Tuttlingen (DE); Deufel, Egon, 78567 Fridingen (DE); Zahler, Sabine, 85591 Vaterstetten (DE)
(74) Vertreter: Witte, Weller & Partner Patentanwälte mbB

(56) Entgegenhaltungen:
- EP-A- 0 306 723
- EP-A- 1 854 415
- EP-A- 2 027 820
- WO-A-2008/020964
- WO-A2-2007/081706
- DE-A1- 3 905 455
- DE-A1- 19 650 721
- US-A- 3 557 780
- US-A1- 2002 165 484

## Beschreibung

Die Erfindung betrifft ein medizinisches Instrument, mit einem Schaft, dessen distales Ende abwinkelbar ist, mit einer am proximalen Ende des Schaftes angeordneten Handhabe, an der eine Abwinkelsteuerung zur Steuerung der Abwinkelung des abwinkelbaren Endes angeordnet ist, wobei die Abwinlzelsteuerung ein verschwenktbares Steuerelement aufweist, dessen Verschwenkung die Abwinkelung verursacht, sowie mit einer Arretierung zum Arretieren der Abwinkelsteuerung in bestimmten Stellungen, wobei das verschwenkbare Steuerelement über ein Reibungselement läuft, und wobei ferner ein Betätigungselement vorhanden ist, über das das Reibungselement aus dem arretierenden Eingriff mit dem Steuerelement bringbar ist.

Ein medizinisches Instrument, entsprechend dem Oberbegriff des Anspruchs 1, ist aus der US 2002/165484 A1 bekannt. Im Inneren der Handhabe ist eine Scheibe angeordnet, die mit Steuerseilen verbunden ist, über die die Abwinkelung des Schaftes steuerbar ist. Ein an der Außenseite der Handhabe angeordnetes Steuerelement ist mit der im Inneren der Handhabe angeordneten Scheibe verbunden. Ein Verschwenken dieses Steuerelementes verursacht ein Drehen der Scheibe im Inneren der Handhabe, wodurch dann die Steuerseile entsprechend bewegt werden. Die Arretierung ist an der umfänglichen Kante der Scheibe realisiert und kann als eine Außenverzahnung ausgebildet sein. Diese Außenverzahnung kann mit einer entsprechenden Innenverzahnung im Inneren des Gehäuses der Handhabe in sperrendem Eingriff treten. Der Zusammenbau aus Scheibe und Steuerelement kann insgesamt gegen eine Feder so verschoben werden, dass die Zähne der Außenverzahnung der Scheibe aus dem sperrenden Eingriff mit der Innenverzahnung am Gehäuse treten. Dadurch ist dann die Verschwenkbarkeit zum Steuern der Abwinkelung des abwinkelbaren Endes frei gegeben. Anstatt einer Arretierung über eine Verzahnung kann diese auch über Materialien mit hohen Reibungskoeffizienten erfolgen, so dass die Arretierung durch ein Reibungselement erfolgt.

Aus der WO 2007/081706 A2 ist ein ähnliches medizinisches Instrument bekannt, bei dem durch Eindrücken des Steuerelementes ein im Inneren des Gehäuses angeordneter Arretiermechanismus, der ebenfalls auf dem Verzahnungsprinzip beruht, gelöst werden kann, um die Verschwenkbarkeit freizugeben. Weitere derartige medizinische Instrumente sind aus der DE 106 50 721 A1, der US 3 557 780 A, der EP 0 306 723 A, der DE 39 05 455 A1, der WO 2008/020964 A2 und der EP 1 854 415 A bekannt.

Die DE 106 50 721 A1 offenbart ein medizinisches Instrument der eingangs genannten Art, wobei das Reibungselement innerhalb des medizinischen Instrumentes angeordnet ist und über einen separaten Hebel in eine arretierende und in eine nicht arretierende Position bewegt werden kann, indem dieser Hebel entlang seiner Längsrichtung verschoben wird. Ähnliche Vorrichtungen zeigen die US 3 557 780 A und die DE 39 05 455 A1, wobei hier der arretierende Zustand durch ein Verdrehen eines entsprechenden Betätigungselementes hergestellt und auch wieder aufgehoben werden kann. Bei den Vorrichtungen der EP 0 306 723 A, der WO 2008/020964 A2 und der EP 1 854 415 A geschieht demgegenüber das Aktivieren bzw. Desaktivieren der Arretierung durch ein Betätigungselement in Form eines kippbaren Hebels. Medizinische Instrumente mit einem abwinkelbaren Ende des Schaftes erlauben dem Operateur wesentlich mehr Handhabungsfreiheitsgrade im Bereich des distalen Endes des Instrumentes.

Bei Instrumenten mit einem starren Schaft, die überwiegend für minimal-invasive Eingriffe eingesetzt werden, eröffnet dies die Möglichkeit, in einem Hohlraum Manipulationen mit dem Instrument vorzunehmen, die außerhalb der Längsachse des Schaftes liegen.

Bei flexiblen Schäften besteht die Möglichkeit, diese über Körperkanäle, beispielsweise die Bronchien, die Speiseröhre, oder den Darm in den Körper einzuführen und durch zusätzliches Abwinkeln des distalen Endes des Schaftes können dann die erwähnten zusätzlichen Freiheitsgrade der Handhabung durchgeführt werden.

Die Handhabungen sind vielerlei, so können es Fass- oder Präpariervorgänge, reine Beobachtungsgänge oder kombiniert mit den zuvor genannten Vorgängen, Koagulationsvorgänge und dergleichen sein.

Zur Steuerung der Abwinklung des Schafts ist eine Abwinkelsteuerung vorhanden, die Steuerseile aufweist, die im abwinkelbaren Bereich des Schaftes an Stellen abseits der Mittellängsachse des Schaftes befestigt sind. Durch Ziehen an einem Steuerseil und Nachschieben eines anderen Steuerseils kann die Krümmung oder Abwinklung des Schaftes ausgelöst werden. Proximalseitig sind die Steuerseile auf einem scheiben- oder trommelartigen Körper aufgebracht und dort fixiert. Ein Drehen dieses Körpers bewirkt ein Ziehen an einem Steuerseil und ein Schieben am anderen Steuerseil.

Zum Drehen der Trommel ist diese mit einem verschwenkbaren Steuerelement verbunden, das beispielsweise mit einem Finger einer Hand, die das medizinische Instrument ergriffen hat, hin- und herverschwenkt werden kann.

Es ist eine Aufgabe der vorliegenden Erfindung ein medizinisches Instrument der eingangs genannten Art dahingehend weiterzuentwickeln, dass der abwirikelbare Abschnitt des Schaftes in jeder beliebigen Stellung arretierbar ist, wobei dies durch einen sicheren und ergonomisch zu handhabenden Steuermechanismus erfolgen soll. Diese dem Anmeldungsgegenstand zugrundeliegende Aufgabe wird durch die im Anspruch 1 angegebenen kennzeichnenden Merkmale gelöst, weitere besondere Ausführungsformen sind in den Unteransprüchen aufgeführt.

Erfindungsgemäß wird die Aufgabe dadurch gelöst, dass das Reibungselement an einer Außenseite der Handhabe und zwischen deren Gehäuse und dem Steuerelement angeordnet ist, dass das Betätigungselement am verschwenkbaren Steuerelement angeordnet ist, dass das Steuerelement ein Fingermuldenteil aufweist und dass das Betätigungselement im Fingerznuldenteil angeordnet ist, und dass das Betätigungselement als Drücker ausgebildet ist, über den das Reibungselement aus dem arretierenden Eingriff vom Steuerelement wegbewegbar ist.

Dadurch, dass das verschwenkbare Steuerelement über ein Reibungselement läuft, kann es in jeder seiner Verschwenkstellungen arretiert werden, sofern es in reibenden Eingriff mit dem Reibungselement steht. Das Reibungselement wirkt also wie eine Art Feststellbremse, die in jedem beliebigen Verschwenkzustand des Steuerelementes zu dem arretierenden Reibschluss führt. Durch das Vorsehen eines zusätzlichen Betätigungselements kann das Reibungselement aus einem arretierenden Eingriff mit dem Steuerelement gebracht werden und das Steuerelement kann dann wieder verschwenkt werden, um eine Änderung des Abwinkelungsmaßes des abwinkelbaren Endes über die Abwinkelsteuerung zu verursachen bzw. zu steuern.

Diese Arretierung ist auch deswegen besonders ergonomisch, da das verschwenkbare Steuerelement und das Reibungselement, ohne eine Betätigung des Betätigungselements, im Reibschluss stehen. Das bedeutet, wird das medizinische Instrument gehandhabt und das Betätigungselement nicht betätigt, ist das Steuerelement arretiert bzw. eine zuvor von diesem verursachte Abwinklung des abwinkelbaren Schaftes wird in dieser abgewinkelten Stellung gehalten. Die Handhabungsperson muss keine Aufmerksamkeit dahingehend entwickeln, dass die Arretierstellung bewerkstelligt wird. Nur wenn eine Veränderung gewünscht ist, muss das Betätigungselement betätigt und dadurch die Arretierung gelöst werden und das Steuerelement kann dann beliebig in seinem Verschwenkungsbereich hin- und herbewegt werden, sei es zum geradlinigen Ausrichten des Schaftes oder um das abwinkelbare Ende in seinem Verschwenkbereich in eine andere Position zu bringen. Nach Freigeben des Betätigungselementes ist dann wieder die Arretierung geschlossen.

Dies erhöht die Sicherheit der Handhabung des medizinischen Instrumentes auch dahingehend, dass in einer bestimmten abgewinkelten Position entsprechende Manipulationen durchgeführt werden können, beispielsweise ein Präpariervorgang oder ein Koagulationsvorgang, ohne dass das Maß der Abwinkelung verändert wird.

Bei der Erfindung ist das Reibungselement an der Handhabe angeordnet.

Diese Maßnahme hat den Vorteil, dass das Reibungselement an einem relativ großen Bauteil des medizinischen Instrumentes befestigt werden kann, somit ein entsprechend großes und massiges Widerlager gegenüber den Reibungskräften vorhanden ist.

Bei der Erfindung ist das Betätigungselement am verschwenkbaren Steuerelement angeordnet. In ergonomischer Hinsicht hat dies den beachtlichen Vorteil, dass mit ein und demselben Element sowohl die Steuerung der Abwinkelsteuerung bewerkstelligt werden kann als auch die Aufhebung bzw. Schließung der Arretierung. Besonderes ergonomisch ist dies deswegen, da ein Verschwenken des Steuerelements zunächst ein Lösen der Arretierung erforderlich macht, da es ansonsten nicht bewegt werden kann, so dass es besonders günstig ist, diese beiden dafür notwendigen Bauelemente, nämlich Betätigungselement und Steuerelement zu vereinen.

Dabei weist das Steuerelement einen Fingermuldenteil auf, und das Betäitigungselement ist im Fingermuldenteil angeordnet.

Diese Ausgestaltung zeigt eine besonders hohe Ergonomie dahingehend, dass ein Finger einer Hand, die das medizinische Instrument hält, in diese Fingermulde eingelegt werden kann und von diesem Finger sowohl das Betätigungselement zum Lösen der Arretierung als auch das Bewegen des Steuerelements zum Abwinkeln des Schaftes durchgeführt werden kann. Je nach Anordnung des Steuerelements am medizinischen Instrument, kann das bspw. der Daumen der Hand sein.

Dabei ist das Betätigungselement als Drücker ausgebildet, über den das Reibungselement aus dem arretierenden Eingriff vom Steuerelement wegbewegbar ist.

Diese Maßnahme hat den Vorteil, dass durch einen einfachen Drückvorgang die Arretierung gelöst und dann das Steuerlement verschwenkt werden kann.

In einer weiteren Ausgestaltung der Erfindung ist das Reibungselement in Richtung des Steuerelementes vorgespannt.

Diese Maßnahme hat den Vorteil, dass die Reibungskraft für den arretierenden Reibschluss durch die Vorspannung des Reibungselementes zur Verfügung gestellt wird, was produktions- und steuerungstechnisch ebenfalls sehr günstig ist. Ist konstruktionsbedingt eine relativ große Rückstellkraft in der Abwinkelsteuerung vorhanden, kann dann die Vorspannung entsprechend stark gewählt werden, um ein sicheres Arretieren zu gewährleisten. Somit kann flexibel auf unterschiedliche Arbeitseinsätze in dem Schaft und auf unterschiedliche Größen und Längen des Schaftes reagiert werden.

In einer weiteren Ausgestaltung der Erfindung ist das Reibungselement als ein Reibungsblech ausgebildet, das über Federbleche in Richtung des Steuerelementes drückbar ist.

Diese Maßnahme hat den Vorteil, dass über das Reibungsblech eine relativ große variable Reiburzgsfläche zur Verfügung gestellt werden kann, über die das verschwenkbare Steuerelement in dessen Verschwenkungsbereich bewegt werden kann. Das Bewerkstelligen der Vorspannung in Richtung des Steuerelementes über Federbleche ist produktionstechnisch sehr einfach durchzuführen, beispielsweise durch entsprechende Stanzteile, die auch einfach montiert bzw. ersetzt werden können.

In einer weiteren Ausgestaltung der Erfindung sind die Federbleche als endseitig am Reibungsblech angeordnete Abwinklungen ausgebildet.

Diese Maßnahmen haben den zuvor erwähnten Vorteil der einfachen Herstellung in besonders ausgeprägtem Maße.

In einer weiteren Ausgestaltung der Erfindung weist das Reibungsblech endseitige Langlochöffnungen auf, über die es an einer Außenseite der Handhabe montierbar ist.

Diese Maßnahme hat den Vorteil, dass das Reibungsblech die Ausweich- oder Verschiebebewegung, insbesondere, wenn dieses gekrümmt ist, über die Langlochöffnungen ausführen kann. Gleichermaßen einfach ist das Reibungsblech über diese Langlachöffnung zu montieren, beispielsweise durch einfache Montageschrauben.

In einer weiteren Ausgestaltung der Erfindung steht eine Unterseite des Steuerelementes über eine Reibkontaktfläche mit dem Reibungselement in Kontakt.

Diese Maßnahme hat den Vorteil, dass die Reibkontaktfläche von der Außenseite her relativ gut geschützt ist und insbesondere andere Seiten des Steuerelementes nicht belegt, so dass an diesem die notwendigen Manipulationen, also das Bewegen und das Steuern der Arretierung ungehindert durchgeführt werden kann.

Durch die zuvor erwähnte Vorspannung des Reibungselementes in Richtung des Steuerelementes wird nach Freigeben des Drückers dieser wieder in die entgegengesetzte Richtung verschoben, so dass sich dann schlicht und einfach durch Freigeben des Drückers die Arretierung wieder von selbst schließt. Dies kann in jeder beliebigen Stellung des Verschwenkbereiches des Steuerelementes erfolgen, selbstverständlich auch in dieser Stellung, in der der Schaft dann wieder geradlinig ausgerichtet ist.

In einer weiteren Ausgestaltung der Erfindung weist der Drücker mindestens einen Stift auf, der auf dem Reibungselement steht.

Diese Maßnahme hat den Vorteil, dass die Kraft zur Lösung der Arretierung über zumindest einen Stift auf das Reibungselement ausgeübt werden kann.

In einer weiteren Ausgestaltung der Erfindung bewegt der zumindest eine Stift das Reibungselement aus dem arretierenden Eingriff von dem Steuerelement weg und gleitet bei einem verschwenkenden Steuerelement über das Reibungselement.

Diese Maßnahme hat den Vorteil, dass der oder die Stifte relativ reibungsarm über das Reibungselement beim Verschwenken des Steuerelementes laufen können und dieses bei gedrücktem Drücker laufend vom Steuerelement wegbewegt halten.

In einer weiteren Ausgestaltung der Erfindung sind die Stifte in Hülsen im Steuerelement führbar.

Diese Maßnahme hat den Vorteil, dass bei Arretierungen mit hohen Arretierkräften die Stifte einwandfrei durch die Hülsen geführt sind, so dass diese relativ dünn ausgebildet werden können, um die Gleitreibungskräfte möglichst gering zu halten.

In einer weiteren Ausgestaltung der Erfindung sind die Stifte zumindest in dem Bereich in dem sie mit dem Reibungselement in Berührung stehen, reibungsarm ausgebildet.

Diese Maßnahme hat den Vorteil, dass der zumindest eine Stift bei eingedrückten Steuerelement reibungsarm über das Reibungselement gleiten kann.

Dazu können die gesamten Stifte aus einem reibungsarmen Kunststoffmaterial bestehen, mit einem solchen überzogen sein, oder auf einem metallischen Grundkörper auf- oder angebracht sein.

In einer weiteren Ausgestaltung der Erfindung weist das Betätigungselement Merkmale auf, die dessen Griffigkeit erhöhen.

Dabei sind die Merkmale besonders vorteilhaft ausgewählt aus Erhöhungen, Vertiefungen, Rillen, Riffelungen, Mulden, Ausstanzungen und dergleichen.

Diese Merkmale vermitteln der Handhabungsperson einen besonders guten haptischen Kontakt zwischen dem Finger und dem Steuerelement, so dass die entsprechenden Steuervorgänge sicher und ergonomisch durchgeführt werden können. Insbesondere bei der zuvor erwähnten Konstruktion mit den Stiften, die bei gelöster Arretierung über das Reibelement laufen, ist diese Reibkraft über diese Merkmale besonders günstig zu überwinden, ohne dass die Gefahr besteht, dass der Finger vom Steuerelement aufgrund hoher Widerstandskräfte abrutscht.

In einer weiteren Ausgestaltung der Erfindung ist das Steuerelement über einen Verbindungsarm mit einer Trommel verbunden, an der Steuerseile befestigt sind, die die Abwinklung des abwinkelbaren Endes des Schaftes bewirken.

Diese an sich bekannte Maßnahme hat den Vorteil, dass die Steuerbewegung des Steuerelementes über den Erfindungsarm ergonomisch und in einer harmonischen Bewegung auf die Trommel übertragen werden kann, die dann wiederum entsprechend harmonisch und ruckfrei die Steuerseile bewegt.

In einer weiteren Ausgestaltung der Erfindung ist ein Ende eines Steuerseils in einer Befestigungsschraube eingeschoben und ist in dieser über eine Fixierschraube fixiert.

Diese Maßnahme hat den Vorteil, dass die Position und Länge eines Steuerseils über die Befestigungsschraube in eine optimale Lage gebracht bzw. korrigiert werden kann, anschließend wird diese Lage über die Fixierschraube in der Befestigungsschraube fixiert.

In einer weiteren Ausgestaltung der Erfindung ist die Befestigungsschraube durch eine Sicherungsschraube ortsfest gesichert.

Diese Maßnahme hat den Vorteil, dass durch die Sicherungsschraube zusätzlich noch der Sitz der Befestigungsschraube an der Trommel gesichert ist. Ferner ist die Montage vereinfacht. Dieses Zusammenwirken der Schrauben lässt prinzipiell auch ein Nachstellen oder ein Nachziehen der Zugseile bzw. der Zugseilspannung bei Inspektionsvorgängen einfach zu. Man kann auch nach einer bestimmten Einstellung die Schrauben durch Verklebungen zusätzlich sichern. Auch dies trägt dazu bei, die Bewegungen des Steuerelements des Betätigungselements ergonomisch sicher und harmonisch zu übertragen.

In einer weiteren Ausgestaltung der Erfindung weist die Trommel eine umfängliche Rille auf, über die die Steuerseile zu den Befestigungsstellen geführt sind.

Diese Maßnahme trägt ebenfalls dazu bei, für eine sichere und dauerhafte Führung der Zugseile im Bereich der Trommel zu führen, so dass die Verschwenkbewegungen ruckfrei und sicher übertragen werden können.

In einer weiteren Ausgestaltung der Erfindung ist die Trommel als Trommelausschnitt ausgebildet.

Diese Maßnahme hat den Vorteil, dass keine komplette Trommel mit einer 360° Umfangsfläche notwendig ist, sondern nur ein Ausschnitt davon, der ausreichend ist, um die Hin- und Herbewegung der an der Trommel befestigten Steuerseile zum Abwinkeln des abwinkelbaren Endes des Schaftes zu steuern.

In einer weiteren Ausgestaltung der Erfindung weist der Trommelausschnitt eine Seitenfläche auf, die etwa einem Viertelkreis entspricht.

Diese Maßnahme hat den Vorteil, dass ein gegenüber einer kompletten Trommel wesentlich kleiner bauendes Bauteil eingesetzt werden kann, um die Steuerseile hin- und herzubewegen. Es wurde festgestellt, dass eine Bewegung etwa in einem Winkelbereich von 90° ausreichend ist, um die Steuerung des abwinkelbaren Endes durchzuführen.

In einer weiteren Ausgestaltung der Erfindung ist zumindest eine Umlenkrolle benachbart zum Trommelausschnitt angeordnet, um ein Steuerseil einer Umfangsfläche des Trommelausschnittes hinzuzufüren.

Diese Maßnahme hat den Vorteil, dass durch die Umlenkrolle die vom Schaft kommenden Seile ausgerichtet der Umfangsfläche des Trommelausschnitts zugeführt werden können. Dadurch ist bei einer schlanken bzw. kompakten Bauweise dennoch eine sichere Steuerung gewährleistet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Die Erfindung wird nachfolgend anhand einiger ausgewählter Ausführungsbeispiele in Zusammenhang mit den beiliegenden Zeichnungen näher beschrieben und erläutert. Es zeigen:
   - Fig. 1: ein medizinisches Instrument mit abwinkelbaren Schaft in Seitenansicht,
   - Fig. 1a: das medizinische Instrument von Fig. 1 aus proximaler Betrachtung, d.h. aus Sicht des Operateurs,
   - Fig. 1b: eine perspektivische Ansicht des medizinischen Instrumentes,
   - Fig. 2: eine ausschnittsweise Seitenansicht des medizinischen Instruments von Fig. 1 von der gegenüberliegenden Seite,
   - Fig. 3: eine ausschnittsweise Seitenansicht des medizinischen Instruments von Fig. 1 mit geöffnetem Gehäuse einer Handhabe,

   - Fig. 4: eine ausschnittsweise Detailansicht der Handhabe zur Erläuterung der Arretierung einer Abwinkelsteuerung,
   - Fig. 4a: die Detailansicht von Fig. 4 mit Schnittansicht eines Steuerelements der Abwinkelsteuerung in arretiertem Zustand,
   - Fig. 5: die Detailansicht wie Fig. 4a, in nicht arretiertem Zustand,
   - Fig. 6: das Steuerelement der Abwinkelsteuerung in perspektivischer Einzelansicht,
   - Fig. 6a: eine Ansicht des Steuerelements von Fig. 6, entlang des Pfeils 93 von Fig. 6,
   - Fig. 7: ein Betätigungselement der Abwinkelsteuerung in perspektivischer Einzelansicht,
   - Fig. 8: ein Reibungselement zur Arretierung der Abwinkelsteuerung in perspektivischer Einzelansicht,
   - Fig. 9: eine Detailansicht wie in Fig. 4 dargestellt, mit Verlauf von Steuerseilen um eine Trommel,
   - Fig. 10: eine Seitenansicht der Trommel von Fig. 9,
   - Fig. 10a: einen Schnitt längs der Linie Xa-Xa in Fig. 10,
   - Fig. 10b: ein weiteres Ausführungsbeispiel einer Trommel in Form eines Trommelausschnittes,
   - Fig. 11: eine Seitenansicht eines Werkzeugeinsatzes für das medizinische Instrument von Fig. 1,

   - Fig. 12: eine vergrößerte Detailansicht des distalen Endes des Werkzeugs von Fig. 11,
   - Fig. 13: Seitenansicht eines Griffteils des in Fig. 1 gezeigten Instruments,
   - Fig. 13a: eine Ansicht des Griffteils in Richtung des Pfeils 134 von Fig. 13 betrachtet,
   - Fig. 14: ein vergrößerter abschnittsweiser Schnitt längs der Linie XIV-XIV in Fig. 13, mit eingehängtem Ende des Werkzeugeinsatzes von Fig. 11 im gesperrten Zustand,
   - Fig. 14a: eine der Fig. 14 entsprechende Darstellung mit freigegebenem Ende des Werkzeugeinsatzes,
   - Fig. 15: ein vergrößerter abschnittsweiser Schnitt längs der Linie XV-XV in Fig. 13a, mit eingehängtem Ende des Werkzeugeinsatzes von Fig. 11,
   - Fig. 16: eine Sperre in perspektivischer Einzelansicht,
   - Fig. 17: eine Detailansicht eines medizinischen Instruments im Bereich des Griffteils zur Veranschaulichung der Rastverbindung,
   - Fig. 18: eine Darstellung entsprechend Fig. 17, mit gelöster Rastverbindung,
   - Fig. 19: eine Darstellung entsprechend Fig. 17, als Schnitt in der Betrachtungsebene und mit deaktivierter Rastverbindung, und
   - Fig. 20: eine Darstellung entsprechend Fig. 19, mit aktivierter Rastverbindung.

Ein in den Figuren dargestelltes medizinisches Instrument ist in seiner Gesamtheit mit der Bezugsziffer 10 bezeichnet.

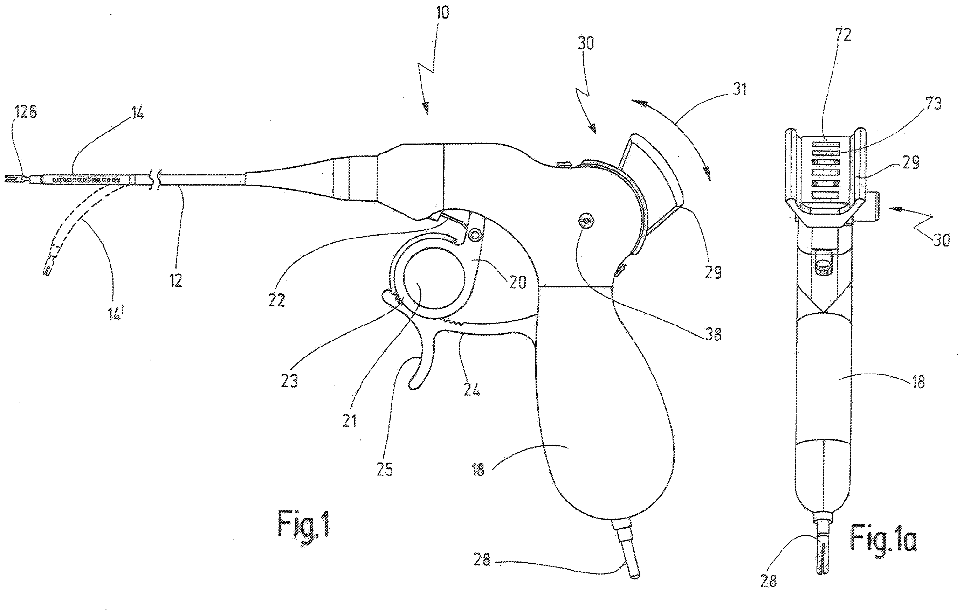

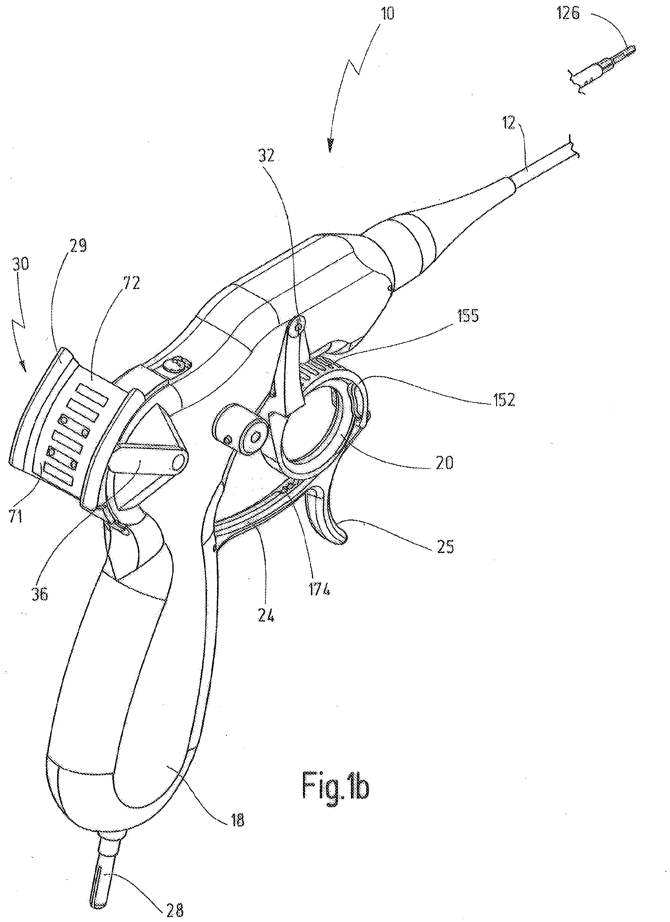

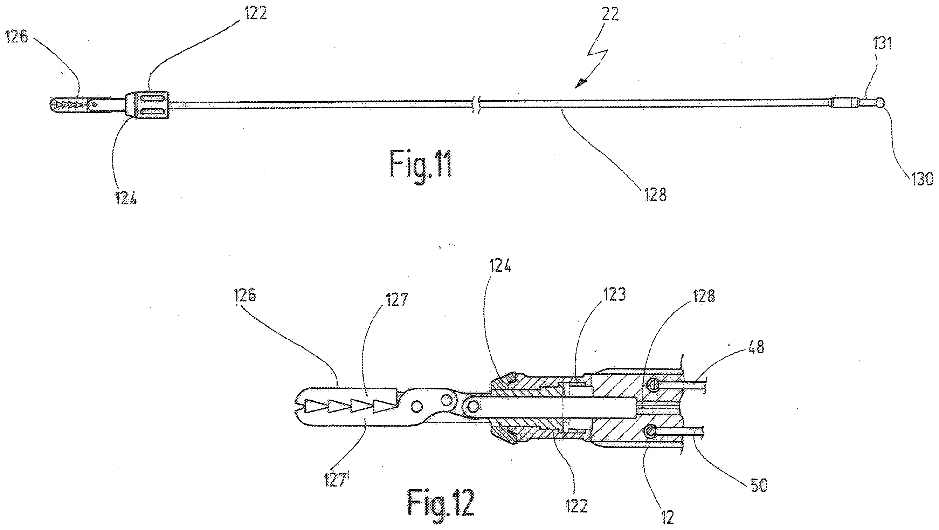

Das in Fig. 1 gezeigte medizinische Instrument 10 weist einen flexiblen Schaft 12 auf, an dessen distalen Ende sich ein abwinkelbarer Bereich 14 befindet. Am Bereich 14 ist distalseitig ein Werkzeug 126 angeordnet. Das Werkzeug 126 stellt ein distales Ende eines in Fig. 11 dargestellten Einsatzes 22 dar. Das proximale Ende des Schaftes 12 ist mit einer Handhabe 18 verbunden.

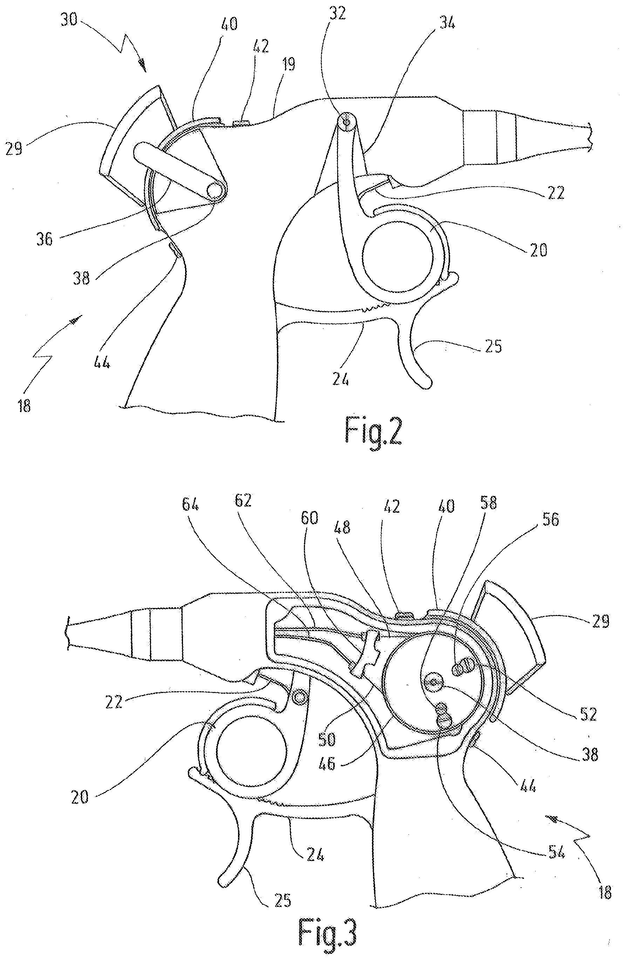

Die Handhabe 18 ihrerseits weist ein bewegliches Griffteil 20 auf. Dieses besitzt eine runde Öffnung 21, die von einem Ringabschnitt 23 umgrenzt ist, durch die vorzugsweise der Zeigefinger des Operateurs geführt werden kann, um eine Bewegung des Griffteils 20, das um die in der Fig. 2 gezeigte Schwenkachse 32 verschwenkbar ist, durchzuführen. Das Griffteil 20 ist mit dem proximalen Ende des Einsatzes 22 verbunden. Durch die Verbindung des Griffteils 20 mit dem Einsatz 22, steht dieser in Wirkverbindung mit dem Werkzeug 126 und dient so zu dessen Betätigung, so z.B. einem Öffnen und Schließen eines Maulteils.

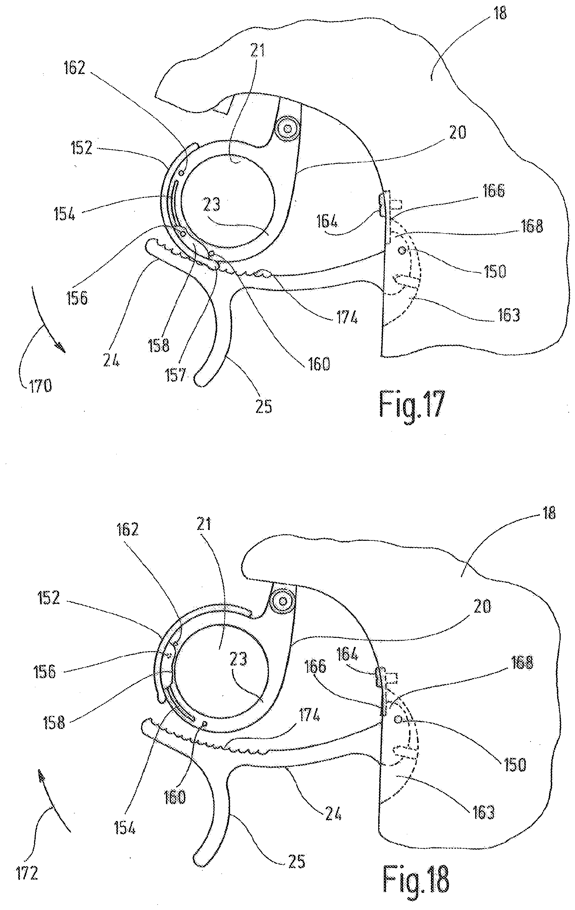

Weiterhin kann das Griffteil 20 in Kontakt mit einer Raste 24 gebracht werden, die eine ungewollte Bewegung des Griffteils 20 in eine distale Richtung verhindern kann. Um eine Lösung der Rastverbindung zu ermöglichen, weist die Raste 24 unter anderem einen bogenförmigen Ansatz 25 auf, der ein Verschwenken der Raste 24 durch den Operateur, vorzugsweise mit dem Mittelfinger, entsprechend der Beschreibung in Zusammenhang mit den Figuren 17ff. ermöglicht.

Des Weiteren ist an der Handhabe 18 ein Steuerelement 29 einer Abwinkelsteuerung 30 angeordnet, durch dessen Bewegung entsprechend der Richtungen des Doppelpfeils 31 um eine Drehachse 38, die senkrecht zur hier dargestellten Achse des Schaftes 12 steht, die Abwinklung des abwinkelbaren Endes 14 des Schaftes 12 gesteuert werden kann. Ein Beispiel für eine Richtung der Abwinklung ist in Fig. 1 durch das abgewinkelte Ende 14' dargestellt.

In Fig. 1a blickt man auf das Steuerelement 29 der Abwinkelsteuerung 30 und ein sich darauf befindliches Betätigungselement 71 in Form eines Drückers 72. Dieser kann durch einen Daumen des Operateurs betätigt werden, wodurch eine Bewegung des Steuerelements 29 ermöglicht wird. Zur besseren Greifbarkeit sind hierfür Rillen 73 auf dem Drücker 72 angebracht.

Das Instrument 10 weist ferner noch einen Stromanschluss 28 auf, der z.B. zur Stromversorgung von optional einsetzbaren Koagulationseinsätzen dienen kann.

Aus Fig. 2 kann man den Bereich der Verschwenkbarkeit des Griffteils 20 um die Schwenkachse 32 im Bereich einer Aussparung 34 erkennen. Das Steuerelement 29 ist über einen Verbindungsarm 36 mit der Drehachse 38 verbunden.

Zwischen einem Gehäuse 19 der Handhabe 18 und dem Steuerelement 29 befindet sich ein Reibungselement 39 in Form eines Reibungsbleches 40, welches durch Schrauben 42 und 44 an der Außenseite der Handhabe 18 befestigt ist. Dieses Reibungsblech 40 dient, wie im Folgenden noch näher beschrieben wird, die Abwinkelsteuerung 30 in einer bestimmten Position zu arretieren.

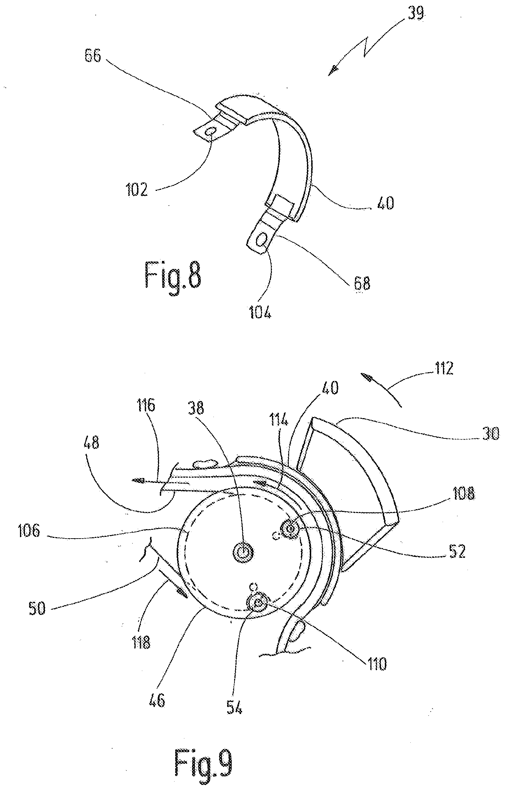

Eine in Fig. 3 erkennbare Trommel 46 ist auf der Drehachse 38 befestigt und steht somit durch den Verbindungsarm 36 mit dem Steuerelement 29 der Abwinkelsteuerung 30 in Wirkverbindung. Eine entsprechende Betätigung des Steuerelements 29 hat somit auch eine direkte Bewegungsübertragung auf die Trommel 46 zur Folge. An der Trommel 46 enden zwei vom abwinkelbaren Ende 14 des Schaftes 12 durch den Schaft 12 verlaufende Steuerseile 48 und 50, die jeweils abseits der Drehachse 38 verlaufen und in diesem Ausführungsbeispiel durch Befestigungsschrauben 52 und 54 in Kombination mit Sicherungsschrauben 56 und 58 an der Trommel 46 befestigt sind. Dazu werden die Steuerseile 48 und 50 von distal her aus Hüllen 62 und 64 austretend durch eine Führung 60 zu der Trommel 46 geleitet. Die Steuerseile 48 und 50 sind die Betätigungselemente für das abwinkelbare Ende 14. Zusammen mit der Trommel 46 und dem Verbindungsarm 36 ergibt sich somit die Wirkverbindung zwischen dem Steuerelement 29 der Abwinkelsteuerung 30 und dem abwinkelbaren Ende 14. Eine detailliertere Funktionsbeschreibung erfolgt später im Zusammenhang mit Fig. 9.

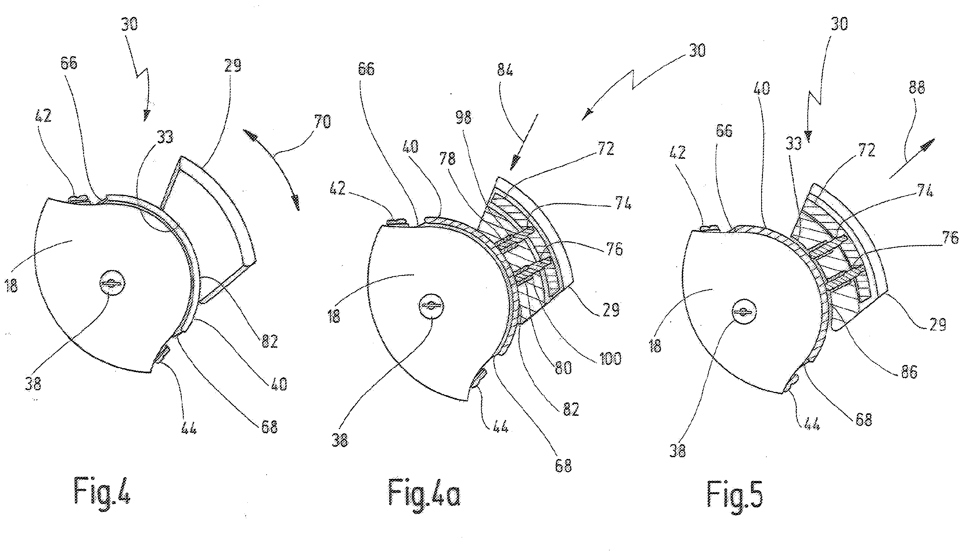

In Zusammenhang mit den Figuren 4 bis 8 wird nunmehr auf die Arretierbarkeit der Abwinkelsteuerung 30 und somit des abwinkelbaren Endes 14 des Schaftes 12 mit Hilfe des Reibungsblechs 40 näher eingegangen.

In Fig. 4 sieht man, dass sich eine Unterseite 33 des Steuerelements 29 in direktem Kontakt mit dem Reibungsblech 40 befindet, welches über abgewinkelte Federbleche 66 und 68 und mittels der Schrauben 42 und 44 an der Handhabe 18 befestigt ist. Das Reibungsblech 40 verläuft somit im Abstand zur Außenseite der Handhabe 18 an der es montiert ist. Eine Bewegung der des Steuerelements 29 um die Drehachse 38 entsprechend der Richtungen des Doppelpfeils 70, wird hierbei durch den reibenden Kontakt zwischen dem Steuerelement 29 und dem Reibungsblech 40 an einer Reibkontaktfläche 82 verhindert, bzw. gebremst.

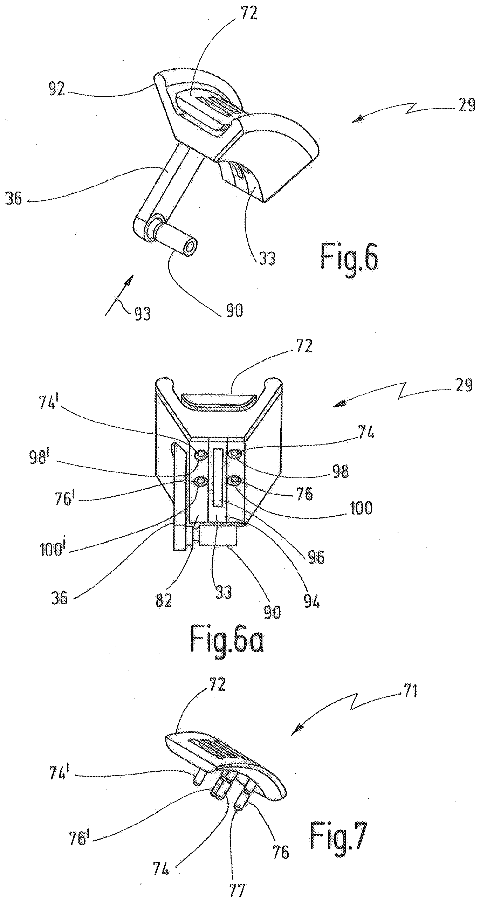

In Fig. 4a ist zu erkennen, dass die Abwinkelsteuerung 30 den Drücker 72 umfasst. Dieser ist, wie auch in Fig. 1b zu erkennen ist, proximalseitig für den Operateur gut zugänglich. Vom Drücker 72 stehen Stifte 74, 74', 76, 76' (siehe auch Fig. 7) vor, die distalseitig durch Hülsen 78, 80 im Körper des Steuerelements 29 geführt und gehalten sind. Die Spitzen der Stifte stehen auf dem Reibungsblech 40 direkt auf und ergeben somit eine Wirkverbindung zwischen dem Drücker 72 und dem Reibungsblech 40. Durch Eindrücken des Drückers 72 entsprechend der Richtung des Pfeils 84, werden die Stifte durch Bohrungen 98, 100 im Körper des Steuerelements 29 axial bewegt, wobei sie das Reibungsblech 40 in Richtung der Handhabe 18 drücken. Das Reibungsblech 40 bewegt sich dadurch von der Unterseite 33 des Steuerelements 29 weg. Die Reibkontaktfläche 82 ist damit gelöst und es ergibt sich ein Spalt 86, wie er in Fig. 5 dargestellt ist. Die für diese Positionsänderung des Reibungsblechs 40 benötigte Flexibilität, wird vor allem durch die Federbleche 66 und 68, aber auch durch Langlochöffnungen 102 und 104, wie sie in Fig. 8 gezeigt sind, ermöglicht.

Die durch Betätigen des Drückers 72 resultierende Stellung, wie sie in Fig. 5 gezeigt ist, erlaubt nun eine reibungsarme Bewegung des Steuerelements 29, wie sie in Fig. 4 anhand des Doppelpfeils 70 gezeigt ist.

Lediglich die Spitzen der vier Stifte 74, 74', 76, 76' stehen auf dem Reibungsblech 40 und gleiten reibungsarm über dessen Oberfläche. Dazu können diese z.B. aus einem reibungsarmen Kunststoffmaterial bestehen. Es kann auch ein metallischer Grundkörper mit dem reibungsarmen Material überzogen sein, oder an einen metallischen Stumpf eine reibungsarme Spitze ausgesetzt sein.

Nimmt nun der Operateur seinen Finger, vorzugsweise den Daumen, wieder vom Drücker 72, so wird aufgrund der Spannung durch die Federbleche 66 und 68 das Reibungsblech 40 wieder gegen die Unterseite 33 des Steuerelements 29 der Abwinkelsteuerung 30 gedrückt, so dass der Spalt 86 verschwindet und wieder die Reibkontaktfläche 82 vorliegt. Entsprechend erfahren die Stifte 74, 74', 76, 76' und damit der Drücker 72 auch wieder eine proximal gerichtete Bewegung entsprechend der Richtung des Pfeils 88. Dadurch wird die Abwinkelsteuerung 30 wieder in ihrer Position arretiert. Dies ist somit im Verschwenkbereich des Steuerelements 29 stufenlos durchzuführen.

Das Steuerelement 29 der Abwinkelsteuerung 30 ist in Fig. 6, 6a und 7 näher dargestellt, wobei man hier deutlich den Drücker 72 und dessen proximale Zugänglichkeit erkennt. Der Drücker 72 ist dabei an einem Fingermuldenteil 92 befestigt, welches über den Verbindungsarm 36 und mit einem Achsbolzen 90 an der Drehachse 38 montiert wird.

Fig. 6a zeigt die Unterseite 33, die mit dem Reibungsblech 40 in Kontakt kommt. In diesem Ausführungsbeispiel ist der Drücker 72 mit vier Stiften 74, 74', 76 und 76' ausgestattet, die axial in den Bohrungen 98, 98', 100 und 100' verlaufen und bewegbar sind. Zwischen den beiden Stiftpaaren 74, 76 und 74', 76' ist ein Kunststoffinlet 96 angeordnet, welches durch ein Halteblech 94 am Fingermuldenteil 92 befestigt wird. Dieses Kunststoffinlet 96 dient dazu, die Reibung zwischen dem Steuerelement 29 und dem Reibungsblech 40 zu erhöhen und somit die Arretierung in der gewünschten Position zu stärken.

In Fig. 7 ist der Drücker 72 mit den vier Stiften 74, 74', 76 und 76' gut zu erkennen. Aufgrund ihrer distal abgerundeten Spitzen 77 wird die Reibung bei ihrem Gleiten auf dem Reibungsblech 40 auf ein Minimum reduziert, was die Bedienbarkeit der Abwinkelsteuerung 30 erleichtert.

Das in Fig. 8 gezeigte Ausführungsbeispiel des Reibungsblechs 40 ist an den gegenüberliegenden Enden mit den abgewinkelten Federblechen 66 und 68 verbunden, die beide eine Langlochöffnung 102 bzw. 104 besitzen, wodurch eine Beweglichkeit des Reibungsblechs 40 an der Handhabe 18, entsprechend der vorherigen Beschreibungen, also auf die Handhabe 18 zu bzw. von dieser weg, ermöglicht wird. Die Abwinklungen an den Federblechen 66 und 68 sorgen für die entsprechende Andrückkraft und somit für die entsprechend feste Arretierung zwischen Steuerelement 29 und Handhabe 18 an der das Reibungsblech 40 montiert ist.

Anhand der Fig. 9 bis Fig. 10a soll die Funktionsweise der Abwinkelsteuerung 30 weiter erläutert und die Befestigung der Steuerseile 48 und 50 an der Trommel 46 beschrieben werden.

In Fig. 9 ist der Verlauf der Steuerseile 48 und 50 in der Trommel 46 dargestellt. Letztere umfasst eine umfängliche Rille 106 in der die Steuerseile 48 und 50 geführt werden, um dann in Bohrungen 108 und 109 der Befestigungsschrauben 52 und 54 zu enden. An diesen sind die Steuerseile 48 und 50 dann fest montiert.

Wird nun das Steuerelement 29 in Richtung des Pfeils 112 bewegt, so erfährt die Trommel 46, durch die zuvor beschriebene Wirkverbindung über den Verbindungsarm 36, eine Drehbewegung um die Drehachse 38, wie sie durch die Richtung des Pfeils 114 angegeben ist. Für die an der Trommel 46 befestigten Steuerseile 48 und 50 bedeutet dies, dass sie ebenfalls eine Bewegung erfahren und zwar wird das Steuerseil 48 in Richtung des Pfeils 116 in den Schaft 12 hineingeschoben und das Steuerseil 50 entsprechend in Richtung des Pfeils 118 aus dem Schaft herausgezogen. Aufgrund der bereits erwähnten Wirkverbindung zwischen den Steuerseilen 48 und 50 mit dem abwinkelbaren Ende 14, wird dieses in seiner Winkelstellung folglich verändert. Dies resultiert in einem Abwinkeln entsprechend der in Fig. 1 durch das abwinkelbare Ende 14' dargestellten Form.

Die entgegengesetzte Bewegung führt wieder zu einem Strecken des Schaftes 12 bzw. einem Abwinkeln, aus der Sicht von Fig. 1, nach oben. Durch Freigabe des Drückers 72 kann die Position bzw. Abwinklung des abwinkelbaren Endes 14 in jeder beliebigen Stellung arretiert werden.

Die Anordnung der Trommel und die des Steuerelements um 90° verdreht, würde anstatt der in Fig. 1 dargestellten Abwinkelebene "oben-unten" eine um die Schaftachse um 90° verdrehte Abwinkelebene "links-rechts" ergeben. Man kann die Steuerseile auch vertauscht anbringen, dann führt z.B. ein Verschieben des Steuerelements "nach vorne" anstatt zu einer Abwinklung nach "unten" zu einer Abwinklung nach "oben".

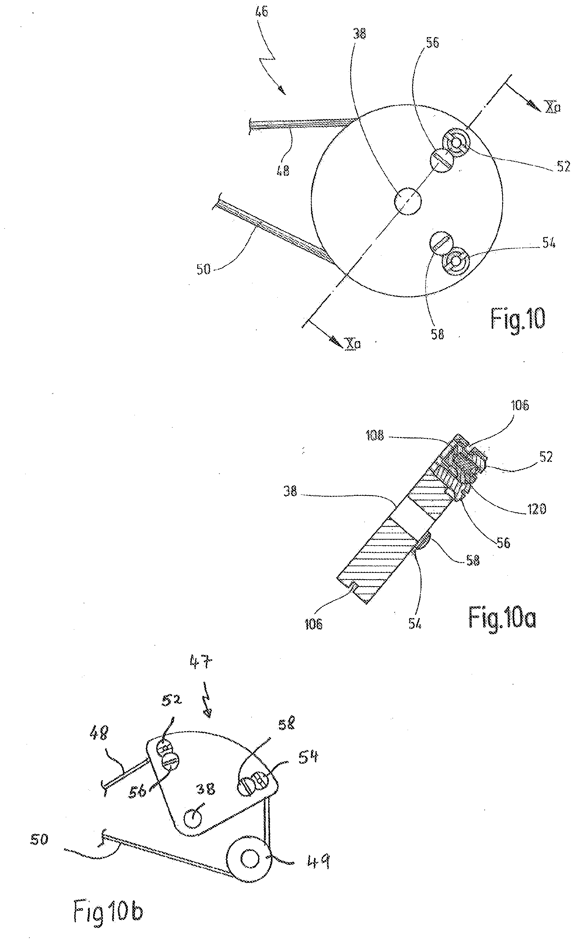

In Fig. 10a erkennt man die Rille 106. Des Weiteren sieht man die Bohrung 108 der Befestigungsschraube 52. Durch diese wird in dem hier erwähnten Beispiel das Steuerseil 48 in die Befestigungsschraube 52 eingeführt und mittels einer Fixierschraube 120 in dieser Befestigungsschraube fest montiert. Entsprechendes gilt für die hier nicht im Querschnitt gezeigte Befestigungsschraube 54 und das Steuerseil 50. Anschließend kann dann die Länge der Steuerseile 48 und 50 durch individuelle Drehung der Schrauben 52 und 54 eingestellt werden. In einem Ausführungsbeispiel haben diese dazu unterschiedlich verlaufende Gewinde, so dass Befestigungsschraube 52 ein Rechts- und Befestigungsschraube 54 ein Linksgewinde aufweist. Nach erfolgter Einstellung der Steuerseile 48 und 50 erfolgt dann die Fixierung der Befestigungsschrauben 52 und 54 mittels der Sicherungsschrauben 56 und 58. Diese verhindern eine eigenständige Drehung der Befestigungsschrauben 52 und 54 und somit ein ungewolltes Verstellen der Steuerseile 48 und 50.

In Fig. 10b ist ein weiteres Ausführungsbeispiel einer Trommel dargestellt. Die Trommel 47 unterscheidet sich von der Trommel 46 dadurch, dass die Trommel 47 ein Ausschnitt darstellt, der etwa einer viertel Trommel 46 entspricht, d.h. deren seitliche Scheibenfläche entspricht einem Kreisausschnitt mit einem Winkel von ca. 90°.

Die Schwenkachse 38 sowie die Befestigung der Steuerseile 48 und 50 über die Befestigungsschrauben 52 und 54 und die Sicherungsschrauben 56 und 58 ist gleich. Für eine gerichtete Zuführung des Steuerseiles 50 ist eine Zuführrolle 49 vorgesehen.

Diese Ausführung baut schlanker, so dass das Instrument 10 insgesamt weniger ausladend im Bereich der Trommel ausgebildet werden kann. Eine Verschwenkung der Trommel 47 in Form des Trommelausschnittes reicht für die Bewegung des abwinkelbaren Endes 14 aus.

In den folgenden Figuren 11 bis 16 wird die Ausgestaltung und die Montage des Einsatzes 22 beschrieben.

Der in Fig. 11 gezeigte Einsatz 22 weist distal ein Werkzeug 126, hier zwei spreizbare Maulteile 127, 127' auf, welches über ein stabförmiges flexibles Betätigungselement 128 mit einem Verbindungsstück 130 in Wirkverbindung steht. Proximal hinter dem Werkzeug 126 ist eine Haube 124 und ein Schraubverschluss 122 angebracht, die beide zur Befestigung des Einsatzes 22 an einem flexiblen Schaft, wie z.B. an den flexiblen Schaft 12 aus Fig. 1, dienen. Das proximale Ende des Einsatzes 22 weist, wie zuvor bereits erwähnt, das Verbindungsstück 130 auf, welches bspw. zur Befestigung an dem Griffteil 20 des medizinischen Instruments 10 dient. Dazu ist dieses in diesem Ausführungsbeispiel das Ende kugelförmig ausgestaltet und proximal hinter einem durchmessergeringerem Abschnitt 131 am Einsatz 22 angeordnet.

Fig. 12 zeigt die Befestigung des distalen Endes des Einsatzes 22 am distalen Ende des Schafts 12. Dabei ist die proximal hinter dem Werkzeug 126 befindliche Haube 124 fest mit dem Einsatz 22 verbunden. Dadurch wird ein Verrutschen des Schraubverschlusses 122 in eine distale Richtung verhindert. Dieser Schraubverschluss 122 wird seinerseits dann auf ein Außengewinde 123 am distalen Ende des Schaftes 12 aufgeschraubt. Das Kraftübertragungselement 128 wird dazu zuvor von distal in den Schaft 12 eingeführt. Durch diese Befestigung ist das distale Ende des Einsatzes 22 lagefixiert. Ein Abwinkeln des abwinkelbaren Endes 14 verursacht dann nicht mehr ein Ausschieben des Einsatzes 22 aus dem distalen Ende des Schafts 12.

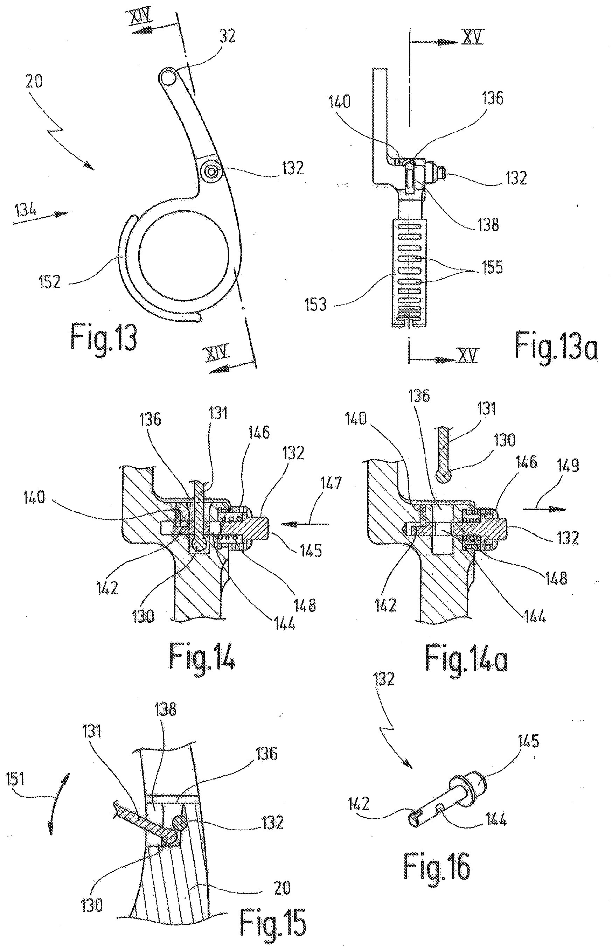

Die Fig. 13 bis 16 zeigen das Griffteil 20, dessen Schwenkachse 32 und einer Sperre 132 zum lösbaren Verbinden mit dem proximalen Ende des Einsatzes 22. In Fig. 13a erkennt man eine in Richtung der Schwenkachse 32 mündende Öffnung 136 durch die das kugelförmige Ende des Verbindungsstücks 130 eingeführt wird. Der distal folgende durchmessergeringe Abschnitt 131 am Einsatz 22 kann über eine Nut 138 seitlich aus dem Inneren des Griffteils 20 herausgeführt werden (siehe Fig. 15). Um das Ende des Einsatzes 22 einzuführen, muss eine Sperre 132 gedrückt werden, so dass das Verbindungsstück 130 diese passieren kann. Dies lässt sich an den Figuren 14 und 14a erkennen.

Die in Fig. 16 ersichtliche Sperre 132 wird durch eine Halterung 146 am Griffteil 20 gehalten. Durch eine Feder 148 wird diese ferner gegen den Rand dieser Halterung 146 gedrückt. Die in Fig. 14 gezeigte Position stellt somit die Grundstellung der Sperre 132 dar. Man sieht hierbei, wie das Verbindungsstück 130 aufgrund seines hier kugelförmigen Endes durch die Sperre 132 blockiert wird und somit nicht mit Bezug auf die Darstellung nach oben durch die Öffnung 136 gelangen kann. Betätigt man nun die Sperre 132 entgegen der Richtung, in der sie durch die Feder 148 gedrückt wird, also in Richtung des Pfeiles 147, so bewegt sich eine Aussparung 144 an der Sperre 132, die sich in der Darstellung von Fig. 14 rechts des Verbindungsstückes 130 befindet, in eine zentrale Position der Öffnung 136, wie sie bspw. in Fig. 14a gezeigt ist. Dieses Eindrücken kann über einen Knopf 145 durchgeführt werden, der seitlich über die Halterung 146 hinaus zur Außenseite ragt. Diese Aussparung 144 gibt dem kugelförmigen Verbindungsstück 130 gerade genügend Platz, um an dieser Sperre 132 vorbeizugelangen. Dadurch kann das Verbindungsstück 130 durch die Öffnung 136 hindurch aus der Halterung in dem Grillteil 20 entfernt werden. Gibt man die Sperre 132 wieder frei, so bewegt sich diese entsprechend der Richtung des Pfeils 149 aus Fig. 14a wieder nach außen. Ursache hierfür ist wiederum die Feder 148. Gleichzeitig verschiebt sich dabei dann auch die Aussparung 144.

Will man das Verbindungsstück 130 nun wieder in die Halterung des Griffteils 20 einsetzen, so bedarf es wieder einem Eindrücken der Sperre 132 in Richtung des Pfeils 147 aus Fig. 14, so dass die Aussparung 144 wieder in die zentrale Lage, wie sie in Fig. 14a gezeigt ist, zu liegen kommt. Auf diese Weise kann man nun das kugelförmige Ende wieder an der Sperre 132 vorbeiführen und das Verbindungsstück 130 durch die Öffnung 136 am Griffteil 20 befestigen.

In Fig. 15 sieht man mit einem Verbindungsstück 130 veranschaulicht, wie sich dieses unterhalb der Sperre 132 befindet. Eine Bewegung nach oben ist nicht möglich. Der durchmessergeringe Abschnitt 131 am proximalen Ende des Einsatzes 22 passt durch die Nut 138 hindurch und somit ist eine Bewegbarkeit entsprechend des Doppelpfeils 151 ermöglicht wird. Diese Bewegungsfreiheit wird bei der Bewegung des Griffteils 20 benötigt.

Um ein Verdrehen der um die Längsachse drehbaren Sperre 132 und damit auch ein Verdrehen der Aussparung 144 zu verhindern, ist am distalen Ende der Sperre 132 eine axiale Nut 142 eingearbeitet. Diese dient auch als Anschlag für die Verschiebebewegung. Dies ist in den Figuren 14 und 14a, sowie auch in der perspektivischen Darstellung der Fig. 16 gezeigt. In dieser Nut 142 endet nun bei der Befestigung im Griffteil 20 ein Stift 140, der zwar ein unerwünschtes Drehen um die Längsachse der Sperre 132 verhindert, eine axiale Beweglichkeit der Sperre 132 in Richtung der Pfeile 147 und 149 jedoch erlaubt.

In den Figuren 17 bis 20 ist die Rastverbindung, die sich durch die Raste 24 auf das Griffteil 20 ergibt, näher erläutert.

Die Raste 24 ist an einer Schwenkachse 150 verschwenkbar an der Handhabe 18 in einer Aussparung 163 angebracht. Durch Kontakt mit dem Griffteil 20 kann diese Raste 24 in diesem Ausführungsbeispiel die Bewegung des Griffteils 20 in die distale Richtung unterbinden. Dazu wird die Raste 24 durch die Vorspannung aufgrund eines Federblechs 166 in Richtung des Griffteils 20 gedrückt.

Die Raste 24 weist dazu an der dem Griffteil 20 zugewandten Seite Rastzähne 174 auf, die mit einem Raststift 160 am Griffteil 20 in Eingriff kommen. Die Neigung der Flanken der Rastzähne 174 in Richtung Handhabe 18, erlauben eine Bewegung des Griffteils 20 in Richtung der Handhabe 18, sperren diese jedoch in der entgegengesetzten Richtung.

Will man die Rastverbindung kurzzeitig lösen, so verschwenkt man die Raste 24 entsprechend der Richtung des Pfeils 170, vorzugsweise durch Betätigung am bogenförmigen Ansatz 25, was zu einer Endstellung, wie sie in Fig. 18 gezeigt ist, führt. Aufgrund der Vorspannung wird die Raste 24 bei Loslassen dieser entsprechend der Richtung des Pfeils 172 wieder zurück an den Griffteil 20 gebracht.

Um für einen bestimmten Zeitraum die Rastverbindung zu deaktivieren, ist am Griffteil 20 eine Rastsperre 152 angebracht.

Die Rastsperre 152 ist als ein gebogenes Element, im dargestellten Ausführungsbeispiel als ein gebogener Streifen 153 ausgebildet (siehe auch Fig. 13a) dessen Krümmung der Krümmung der Außenseite des Ringabschnittes 23 des Griffteils 20 angepasst ist.

Aussparungen bzw. Austanzungen 155 im Streifen 153 erhöhen die Griffigkeit.

Diese Rastsperre 152 kann, wie aus Fig. 17 ersichtlich, zwischen Griffteil 20 und Raste 24 gebracht werden. In diesem Fall ist die Rastverbindung deaktiviert und das Griffteil 20 ist frei in beide Richtungen bewegbar. Dazu weist die Rastsperre 152 eine abgerundete Backe 157 auf, die in beiden Richtungen über die Zähne 174 laufen kann. Dies entspricht einer zweiten Stellung der Rastsperre 152. Um die Rastverbindung nun wieder zu aktivieren, kann die Rastsperre 152 in Richtung eines Raststifts 162 geschoben werden. Dies entspricht einer ersten Stellung der Rastsperre 152. Auf beiden Seiten des Streifens 153 ist eine Abdeckung 158 vorhanden. Diese deckt einen in der Rastsperre quer verlaufenden Führungsstift 156 ab, der in beidseitigen Führungsnuten 154 im Ringabschnitt 23 läuft. Die Abdeckungen 158 selbst können über hier nicht näher dargestellte Stifte an der Rastsperre 152 befestigt werden. Dementsprechend durchläuft die Rastsperre 152 eine kreisförmige Bewegung, wie sie durch die Form des Ringabschnitts 23 des Griffteils 20 vorgegeben ist und endet somit in einer Position, wie sie in Fig. 18 gezeigt ist. Dadurch liegt dann ein zuvor durch die Rastsperre 152 blockierter Raststift 160 frei und kann mit den Zähnen 174 der Raste 24 in Eingriff treten.

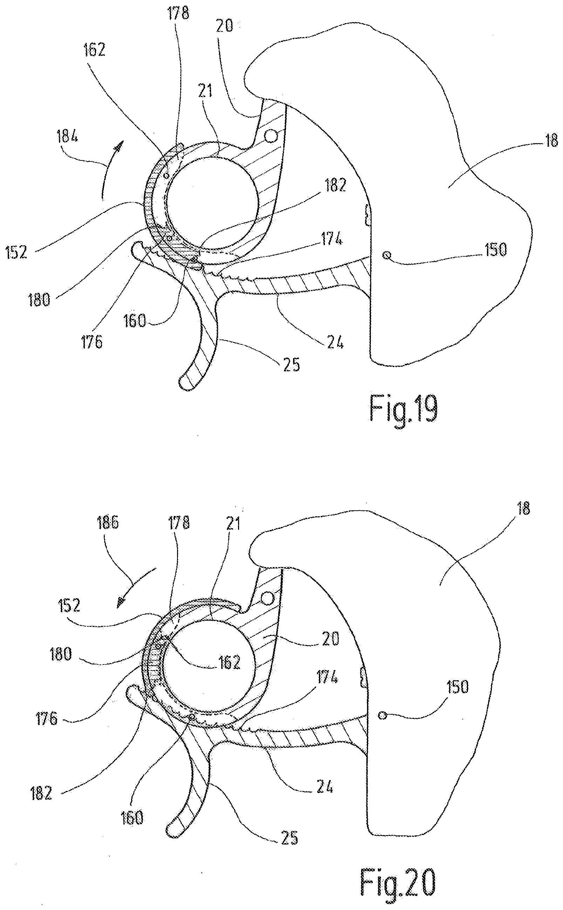

Wie in den Figuren 19 und 20 gezeigt ist, ist umfänglich im Griffteil 20 eine äußere Nut 178 eingeschnitten. In dieser ist eine in radialer Richtung des Ringabschnittes 23 vorspringende Stufe 176 der Rastsperre 152 bewegbar, die mittig an der Rastsperre 152 angeordnet ist. An jeweils einem Ende dieser Stufe 176 befinden sich Federclips 180 bzw. 182. Diese können entsprechend der Position der Rastsperre 152 in den Raststift 160 bzw. 162 einrasten und damit ein einfaches Hin- und Herrutschen der Rastsperre 152 verhindern. Diese wird somit in den jeweiligen Stellungen gehalten.

Fig. 19 zeigt in diesem Zusammenhang die zweite Stellung der Rastsperre 152, in der die Rastverbindung deaktiviert ist. Der Federclip 182 der Stufe 176 an der Rastsperre 152 ist in den Raststift 160 eingerastet, und blockiert somit den Kontakt zwischen den Rastzähnen 174 und dem Raststift 160. Eine Bewegung der Rastsperre 152 in Richtung des Pfeils 184 würde schließlich in der ersten Stellung enden, wie sie in Fig. 20 gezeigt ist. Dabei ist der Federclip 180, der sich an der Stufe 176 befindet, in den Raststift 162 eingerastet, und damit ist die Rastsperre 152 in dieser Position fixiert. Der Raststift 160 liegt somit frei und kann in die Zähne 174 der Raste 24 einhaken.

Dahingegen ist eine proximale Bewegung des Griffteils 20, die z.B. zu einem Schließen der Maulteile 127, 127' führen würde, über die Raste 24 weiter möglich.

Eine Deaktivierung der Rastverbindung kann nun wieder durch Bewegung der Sperre 152 analog zu dem zuvor gesagten entsprechend der Richtung des Pfeils 186, vorzugsweise nach vorherigem Absenken der Raste 24, gemäß der Beschreibung zu den Figuren 17 und 18, erfolgen.

Die Handhabungsperson kann das Instrument 10, wie in Fig. 1a ersichtlich, über die Handhabe 18 ergreifen. Mit dem Daumen kann der Drücker 72 gedrückt werden und dann kann das Steuerelement 29 verschoben werden. Dies bewirkt ein entsprechendes Abwinkeln des abwinkelbaren Endes 14 des Schafts. Ein Freigeben des Drückers 72 arretiert das abwinkelbare Ende 14 in der entsprechenden Stellung.

Ein Bewegen des Griffteils 20, z.B. mit dem eingeschobenen Zeigerfinger, erlaubt das Öffnen und Schließen der Maulteile 127, 127' über den Einsatz 22 in jeder beliebigen Abwinkelstellung des abwinkelbaren Endes 14 des Schaftes 12.

Bei deaktivierter Rastfunktion ist die Bewegung des Griffteils 20 in beiden Schwenkrichtungen möglich.

Bei aktivierter Rastfunktion kann diese kurzzeitig durch Verschwenken der Raste 24 mit dem Mittelfinger über den bogenförmigen Ansatz 25 bewerkstelligt werden.

Die Handhabungsperson kann somit auf sehr ergonomische Art und Weise das medizinische Instrument 10 einfach und sicher bedienen.

## Patentansprüche

1. Medizinisches Instrument, mit einem Schaft (12), dessen distales Ende (14) abwinkelbar ist, mit einer am proximalen Ende des Schaftes (12) angeordneten Handhabe (18), an der eine Abwinkelsteuerung (30) zur Steuerung der Abwinkelung des abwinkelbaren Endes (14) angeordnet ist, wobei die Abwinkelsteuerung (30) ein verschwenkbares Steuerelements (29) aufweist, dessen Verschwenkung die Abwinkelung verursacht, sowie mit einer Arretierung zum Arretieren der Abwinkelsteuerung (30) in bestimmten Stellungen, wobei das verschwenkbare Steuerelement (29) über ein Reibungselernent (39) läuft, und ein Betätigungselement (71) vorhanden ist, über das das Reibungselement (39) aus einem arretierenden Eingriff mit dem Steuerelement (29) bringbar ist, **dadurch gekennzeichnet, dass**
dass das Reibungselement (39) an einer Außenseite der Handhabe (18) und zwischen deren Gehäuse und dem Steuerelement (29) angeordnet ist,
dass das Betätigungselement (71) am verschwenkbaren Steuerelement (29) angeordnet ist,
dass das Steuerelement (29) eine Fingermuldenteil (92) aufweist, und dass das Betätigungselement (71) im Fingermuldenteil (92) angeordnet ist, und
dass das Betätigungselement (71) als Drücker (72) ausgebildet ist, über den das Reibungselement (39) aus dem arretierenden Eingriff vom Steuerelement (29) wegbewegbar ist

2. Medizinisches Instrument nach Anspruch 1, **dadurch gekennzeichnet, dass** das Reibungselement (39) in Richtung des Steuerelementes (29) vorgespannt ist.

3. Medizinisches Instrument nach Anspruch 1 oder 2, **dadurch gekennzeichnet, dass** das Reibungselement (39) als ein Reibungsblech (40) ausgebildet ist, das über Federbleche (66, 68) in Richtung des Steuerelementes (29) drückbar ist.

4. Medizinisches Instrument nach Anspruch 3, **dadurch gekennzeichnet, dass** die Federbleche (66, 68) als endseitig am Reibungsblech (40) angeordnete Abwinkelungen ausgebildet sind.

5. Medizinisches Instrument nach Anspruch 3 oder 4, **dadurch gekennzeichnet, dass** das Reibungsblech (40) endseitige Langlochöffnungen (102, 104) aufweist, über die es an einer Außenseite der Handhabe (18) montiert ist.

6. Medizinisches Instrument nach einem der Ansprüche 1 bis 5, **dadurch gekennzeichnet, dass** eine Unterseite (33) des Steuerelementes (29) über eine Reibkontaktfläche (82) mit dem Reibungselement (39) in Kontakt steht.

7. Medizinisches Instrument nach Anspruch 1, **dadurch gekennzeichnet, dass** der Drücker (72) zumindest einen Stift (74, 74', 76, 76') aufweist, der auf dem Reibungselement (39) steht.

8. Medizinisches Instrument nach Anspruch 7, **dadurch gekennzeichnet, dass** der zumindest eine Stift (74, 74', 76, 76') das Reibungselement (39) aus dem arretierenden Eingriff von dem Steuerelement (29) wegbewegt und bei einem Verschwenken des Steuerelementes (29) über das Reibungselement (39) gleitet.

9. Medizinisches Instrument nach Anspruch 7 oder 8, **dadurch gekennzeichnet, dass** jeder Stift (74, 74', 76, 76') in einer Hülse (78, 80) im Steuerelement (29) führbar ist.

10. Medizinisches Instrument nach einem der Ansprüche 7 bis 9, **dadurch gekennzeichnet, dass** der Stift (74, 74', 76, 76') zumindest in dem Bereich, in dem er mit dem Reibungselement (39) in Berührung steht, reibungsarm ausgebildet ist.

11. Medizinisches Instrument nach einem der Ansprüche 1 bis 10, **dadurch gekennzeichnet, dass** das Betätigungselement (71) Merkmale aufweist, die dessen Griffigkeit erhöhen.

12. Medizinisches Instrument nach Anspruch 11, **dadurch gekennzeichnet, dass** die Merkmale ausgewählt sind aus Erhöhungen, Vertiefungen, Rillen (73), Riffelungen, Mulden, Ausstanzungen.

13. Medizinisches Instrument nach einem der Ansprüche 1 bis 12, **dadurch gekennzeichnet, dass** das Steuerelement (29) über einen Verbindungsarm (36) mit einer Trommel (46, 47) verbunden ist, an der Steuerseile (48, 50) befestigt sind, die die Abwinklung des abwinkelbaren Endes (14) des Schaftes (12) bewirken.

14. Medizinisches Instrument nach Anspruch 13, **dadurch gekennzeichnet, dass** ein Ende eines Steuerseiles (48, 50) in einer Befestigungsschraube (54, 54') eingeschoben ist und in dieser über eine Fixierschraube (120) fixiert ist.

15. Medizinisches Instrument nach Anspruch 14, **dadurch gekennzeichnet, dass** die Befestigungsschraube (52, 54) durch eine Sicherungsschraube (58, 60) ortsfest gesichert ist.

16. Medizinisches Instrument nach einem der Ansprüche 13 bis 15, **dadurch gekennzeichnet, dass** die Trommel (46, 47) eine umfängliche Rille (106) aufweist, über die die Steuerseile (48, 50) zu den Befestigungsstellen führbar sind.

17. Medizinisches Instrument nach einem der Ansprüche 13 bis 16, **dadurch gekennzeichnet, dass** die Trommel (47) als Trommelausschnitt ausgebildet ist.

18. Medizinisches Instrument nach Anspruch 17, **dadurch gekennzeichnet, dass** der Trommelausschnitt eine Seitenfläche aufweist, die etwa einem Viertelkreis entspricht.

19. Medizinisches Instrument nach Anspruch 17 oder 18, **dadurch gekennzeichnet, dass** zumindest eine Umlenkrolle (49) benachbart zum Trommelausschnitt angeordnet ist, um ein Steuerseil (48; 50) einer Umfangsfläche des Trommelausschnitts zuzuführen.

## Claims

1. A medical instrument comprising a shaft (12) whose distal end (14) is bendable, a handle (18) which is arranged at the proximal end of the shaft (12) and on which an angulation control mechanism (30) for controlling the angulation of the bendable end (14) is arranged, the angulation control mechanism (30) having a pivotable control element (29) whose pivoting causes the angulation, and a locking mechanism for locking the angulation control mechanism (30) in defined positions, wherein the pivotable control element (29) runs over a friction element (39), and wherein an actuating element (71) is provided via which the friction element (39) can be brought out of a locking engagement with the control element (29),
**characterized in that**
the friction element (39) is arranged at an outside of the handle (18) and between the housing thereof and the control element (29),
the actuating element (71) is arranged at the pivotable control element (29),
the control element (29) has a finger-receiving part (92) and the actuating element (71) is arranged in the finger-receiving part (92), and
the actuating element (71) is designed as a push piece (72) via which the friction element (39) can be moved out of the locking engagement away from the control element (29).

2. The medical instrument according to claim 1, **characterized in that** the friction element (39) is pretensioned in the direction of the control element (29).

3. The medical instrument according to claim 1 or 2, **characterized in that** the friction element (39) is designed as a friction plate (40) which can be pressed via spring plates (66, 68) in the direction of the control element (29).

4. The medical instrument according to claim 3, **characterized in that** the spring plates (66, 68) are designed as angled pieces arranged at the ends of the friction plate (40).

5. The medical instrument according to claim 3 or 4, **characterized in that**, at its ends, the friction plate (40) comprises elongated openings (102, 104) via which it is mounted at an outside of the handle (18).

6. The medical instrument according to any one of claims 1 to 5, **characterized in that** an underside (33) of the control element (29) is in contact with the friction element (39) via a friction contact face (82).

7. The medical instrument according to claim 1, **characterized in that** the push piece (72) has at least one pin (74, 74', 76, 76') which stands on the friction element (39).

8. The medical instrument according to claim 7, **characterized in that** the at least one pin (74, 74', 76, 76') moves the friction element (39) out of the locking engagement away from the control element (29) and slides over the friction element (39) during a pivoting of the control element (29).

9. The medical instrument according to claim 7 or 8, **characterized in that** each pin (74, 74', 76, 76') is arranged to be be guided in a sleeve (78, 80) in the control element (29).

10. The medical instrument according to any one of claims 7 to 9, **characterized in that** the pin (74, 74', 76, 76') is designed with low friction at least in the area in which it is in contact with the friction element (39).

11. The medical instrument according to any one of claims 1 to 10, **characterized in that** the actuating element (71) has features that increase its grip.

12. The medical instrument according to claim 11, **characterized in that** the features are chosen from elevations, depressions, grooves (73), flutings, hollows, punches.

13. The medical instrument according to any one of claims 1 to 12, **characterized in that** the control element (29) is connected via a connecting arm (36) to a drum (46, 47) on which control wires (48, 50) are fastened which effect the angulation of the bendable end (14) of the shaft (12).

14. The medical instrument according to claim 13, **characterized in that** one end of a control wire (48, 50) is pushed into a fastening screw (54, 54') and is fixed in the latter by a fixing screw (120).

15. The medical instrument according to claim 14, **characterized in that** the fastening screw (52, 54) is secured in place by a securing screw (58, 60).

16. The medical instrument according to any one of claims 13 to 15, **characterized in that** the drum (46, 47) has a circumferential groove (106) via which the control wires (48, 50) can be guided to the fastening sites.

17. The medical instrument according to any one of claims 13 to 16, **characterized in that** the drum (46) is arranged as a drum section.

18. The medical instrument according to claim 17, **characterized in that** the drum section comprises a lateral surface that corresponds approximately to a quarter of a circle.

19. The medical instrument according to claim 17 or 18, **characterized in that** at least one guide roller (49) is arranged adjacent to the drum section for guiding a control wire (48; 50) to a circumferential surface of the drum section.

## Revendications

1. Instrument médical présentant
une tige (12) dont l'extrémité distale (14) peut être coudée,
une poignée (18) disposée à l'extrémité proximale de la tige (12) et sur laquelle est disposée une commande de coudage (30) qui commande le coudage de l'extrémité coudable (14),
la commande de coudage (30) présentant un élément pivotant de commande (29) dont le pivotement provoque le coudage, ainsi qu'un arrêt qui arrête la commande de coudage (30) en différentes positions,
l'élément pivotant de commande (29) passant sur un élément de frottement (39) et un élément d'actionnement (71) par lequel l'élément de frottement (39) peut être amené hors d'un engagement de blocage de l'élément de commande (29) étant prévu, **caractérisé en ce que**
l'élément de frottement (39) est disposé sur le côté extérieur de la poignée (18) et entre son boîtier et l'élément de commande (29),
**en ce que** l'élément d'actionnement (71) est disposé sur l'élément pivotant de commande (29),
**en ce que** l'élément de commande (29) présente une partie (92) en gouttière pour le doigt,
**en ce que** l'élément d'actionnement (71) est disposé dans la partie (92) en gouttière pour le doigt et
**en ce que** l'élément d'actionnement (71) est configuré comme poussoir (72) par lequel l'élément de frottement (39) peut être extrait de l'engagement de blocage de l'élément de commande (29).

2. Instrument médical selon la revendication 1, **caractérisé en ce que** l'élément de frottement (39) est précontraint en direction de l'élément de commande (29).

3. Instrument médical selon les revendications 1 ou 2, **caractérisé en ce que** l'élément de frottement (39) est configuré comme tôle de frottement (40) qui peut être repoussée en direction de l'élément de commande (29) par l'intermédiaire de tôles élastiques (66, 68).

4. Instrument médical selon la revendication 3, **caractérisé en ce que** les tôles élastiques (66, 68) sont configurées comme coudes disposés du côté de l'extrémité de la tôle de frottement (40).

5. Instrument médical selon les revendications 3 ou 4, **caractérisé en ce que** la tôle de frottement (40) présente à son extrémité des ouvertures (102, 104) en trous oblongs par lesquels elle est montée sur un côté extérieur de la poignée (18).

6. Instrument médical selon l'une des revendications 1 à 5, **caractérisé en ce qu'**un côté inférieur (33) de l'élément de commande (29) est en contact avec l'élément de frottement (39) par l'intermédiaire d'une surface (82) de contact par frottement.

7. Instrument médical selon la revendication 1, **caractérisé en ce que** le poussoir (72) présente au moins une tige (74, 74', 76, 76') située sur l'élément de frottement (39).

8. Instrument médical selon la revendication 7, **caractérisé en ce que** la ou les tiges (74, 74', 76, 76') éloignent l'élément de frottement (39) hors de l'engagement de blocage de l'élément de commande (29) et glissent au-dessus de l'élément de frottement (39) lors d'un pivotement de l'élément de commande (29).

9. Instrument médical selon les revendications 7 ou 8, **caractérisé en ce que** chaque tige (74, 74', 76, 76') peut être insérée dans une douille (78, 80) de l'élément de commande (29).

10. Instrument médical selon l'une des revendications 7 à 9, **caractérisé en ce que** la tige (74, 74', 76, 76') présente peu de frottement au moins dans sa partie dans laquelle elle est en contact avec l'élément de frottement (39).

11. Instrument médical selon l'une des revendications 1 à 10, **caractérisé en ce que** l'élément d'actionnement (71) présente des caractéristiques qui renforcent sa saisie.

12. Instrument médical selon la revendication 11, **caractérisé en ce que** les caractéristiques sont sélectionnées parmi des reliefs, des creux, des rainures (73), des cannelures, des moulures et des estampages.

13. Instrument médical selon l'une des revendications 1 à 12, **caractérisé en ce que** l'élément de commande (29) est relié par un bras de liaison (36) à un tambour (46, 47) sur lequel sont fixés des câbles de commande (48, 50) qui ont pour effet le coudage de l'extrémité coudable (14) de la tige (12).

14. Instrument médical selon la revendication 13, **caractérisé en ce qu'**une extrémité d'un câble de commande (48, 50) est insérée dans une vis de fixation (54, 54') et est fixée dans cette dernière par une vis de fixation (120).

15. Instrument médical selon la revendication 14, **caractérisé en ce que** la vis de fixation (52, 54) est immobilisée par une vis de blocage (58, 60).

16. Instrument médical selon l'une des revendications 13 à 15, **caractérisé en ce que** le tambour (46, 47) présente une rainure périphérique (106) par laquelle les câbles de commande (48, 50) peuvent être guidés vers les emplacements de fixation.

17. Instrument médical selon l'une des revendications 13 à 16, **caractérisé en ce que** le tambour (47) est configuré comme section de tambour.

18. Instrument médical selon la revendication 17, **caractérisé en ce que** la section de tambour présente une surface latérale qui correspond sensiblement à un quart de cercle.

19. Instrument médical selon les revendications 17 ou 18, **caractérisé en ce qu'**au moins un rouleau de renvoi (49) est disposé au voisinage de la section de tambour de manière à amener un câble de commande (48; 50) vers une surface périphérique de la section de tambour.
